(19) Europäisches Patentamt
European Patent Office
Office européen des brevets

(11) Veröffentlichungsnummer: **0 084 652**
**B1**

(12) # EUROPÄISCHE PATENTSCHRIFT

(45) Veröffentlichungstag der Patentschrift: **17.04.85**

(51) Int. Cl.⁴: **C 07 D 273/04**, A 01 N 47/38

(21) Anmeldenummer: **82111710.8**

(22) Anmeldetag: **16.12.82**

(54) **Oxadiazindione, Verfahren zu ihrer Herstellung und ihre Verwendung zur Bekämpfung von Insekten und Spinnentieren.**

(30) Priorität: **07.01.82 DE 3200196**

(43) Veröffentlichungstag der Anmeldung: **03.08.83 Patentblatt 83/31**

(45) Bekanntmachung des Hinweises auf die Patenterteilung: **17.04.85 Patentblatt 85/16**

(84) Benannte Vertragsstaaten: **BE CH DE FR GB IT LI NL**

(56) Entgegenhaltungen:
**EP-A-0 014 881**
**DE-A-2 732 115**
**FR-A-2 283 133**

(73) Patentinhaber: **BASF Aktiengesellschaft, Carl-Bosch-Strasse 38, D-6700 Ludwigshafen (DE)**

(72) Erfinder: **Lange, Arno, Dr., Friedrichsplatz 11, D-6800 Mannheim 1 (DE)**
Erfinder: **Kiehs, Karl, Dr., Sudetenstrasse 22, D-6840 Lampertheim 1 (DE)**
Erfinder: **Adolphi, Heinrich, Dr., Kalmitweg 11, D-6703 Limburgerhof (DE)**

**EP 0 084 652 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue Oxadiazindione, Verfahren zu ihrer Herstellung und insektizide und akarizide Mittel, die diese Verbindungen als Wirkstoffe enthalten.

Es ist aus den DE-OS 2 905 687 und 2 732 115 bekannt, daß Oxadiazindione insektizid wirksam sind.

Es wurde gefunden, daß Oxadiazindione der allgemeinen Formel I

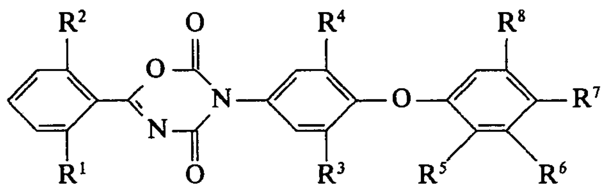

(I)

in der

$R^1$ für $CH_3$, F, Cl, Br und
$R^2$ für H, F, Cl und
$R^3$ bis $R^8$ für H, F, Cl, Br, $CH_3$, $CF_3$, $-OCF_3$, $-OCHF_2$, $-OCF_2CHClF$, $-OCH_3$ oder $-OC_2H_5$ stehen,

insektizid und akarizid besonders wirksam sind.

Bevorzugt sind Verbindungen, bei denen $R^1 = R^2 =$ F oder $R^1 = R^2 =$ Cl oder $R^1 =$ Cl und $R^2 =$ F oder $R^1 =$ Cl und $R^2 =$ H ist.

Bei den Substituenten $R^3$ bis $R^8$ sind folgenden Kombinationen bevorzugt:

a) Wenn $R^3$ und $R^4$ Chlor oder Brom bedeuten, bedeuten mindestens drei der Reste $R^5$ bis $R^8$ Wasserstoff, ein Rest kann Halogen sein;
b) Wenn $R^3$ Wasserstoff und $R^4$ ein F-, Cl- oder Br-Atom oder die Gruppe $CF_3$ bedeutet, sind von den Resten $R^3$ bis $R^8$ mindestens zwei Reste Wasserstoff. Mindestens einer der Reste $R^3$ bis $R^8$ ist F, Cl, Br, $CF_3$, $OCF_3$ oder $OCHF_2$;
c) Wenn $R^3$ und $R^4$ Wasserstoff sind, bedeuten von den Resten $R^5$ bis $R^8$ mindestens zwei Reste F, Br, $OCF_3$ oder $OCHF_2$ bzw. mindestens ein Reste Cl oder $CF_3$.

Man erhält Oxadiazindione (I) durch Umsetzung eines entsprechenden Benzoylisocyanats der allgemeinen Formel II

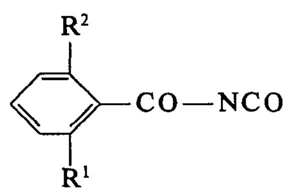

(II)

mit einem entsprechenden Isocyanat der allgemeinen Formel III

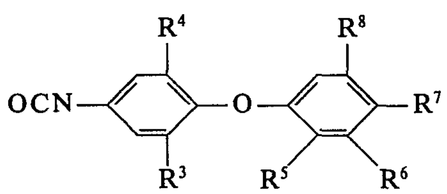

(III)

in an sich bekannter Weise.

Die Reaktionspartner können in etwa stöchiometrischer Menge verwendet werden. Die Reaktionstemperatur liegt im allgemeinen unterhalb von 160°C, vorzugsweise zwischen 80 und 120°C. Vorteilhaft wird ein Katalysator, wie Triethylamin oder eine Zinnverbindung wie Dibutylzinndiacetat, in wirksamer Menge zugesetzt.

Nebenreaktionen der Isocyanate können durch einen Inhibitor wie Phosphortrichlorid verhindert werden.

In den Fällen, wo man ein Lösungsmittel verwenden will, kann man unter den Reaktionsbedingungen chemisch indifferente Stoffe, z. B. aliphatische und aromatische, gegebenenfalls chlorierte oder nitrierte Kohlenwasserstoffe, wie Benzol, Toluol, Xylole, Benzin, Chlorbenzole, 1,2-Dichlorethan, Methylenchlorid, Chloroform, Nitromethan; Tetrachlorkohlenstoff, cyclische und acyclische Ether, wie

Diethylether, Dibutylether, Tetrahydrofuran, Dioxan; acyclische und cyclische Ketone, wie Aceton, Methylethylketon, Methylisopropylketon, Cyclohexanon; Nitrile, wie Acetonitril, Benzonitril und deren Gemische verwenden.

Die Umsetzung verläuft im allgemeinen bei atmosphärischem Druck und kann absatzweise oder fortlaufend vorgenommen werden. Sie verläuft praktisch quantitativ.

Die Oxadiazindione (I) fallen in fester Form an und sind in der Regel für den beabsichtigten Zweck genügend rein; andernfalls können sie durch Umkristallisieren oder Waschen mit einem Lösungsmittel, wie Ether, Petrolether, Cyclohexan oder Toluol, gereinigt werden. Zu ihrer Charakterisierung dienen Elementaranalyse, Schmelzpunkt, IR- und NMR-Spektren.

Die Herstellung der Benzoylisocyanate (II) kann z. B. nach den Vorschriften in J. Org. Chem. 28, 1805–1811 (1963) oder J. Agr. Food Chem. 21, 348 (1973) geschehen.

Ebenso ist die Herstellung der Phenylisocyanate nach bekannten Methoden möglich (vgl. z. B.Weygand-Hilgetag, Organisch-Chemische Experimentierkunst, 1970 oder DE-OS 2 538 178). Für die Herstellung der Diphenylether kann man auf die Angaben z. B. bei Barry et al., Pr. Irish Acad. 53 B, 61, 66, 82 (1950) zurückgreifen.

## Herstellungsbeispiel

a) 9,2 g 2,6-Difluorbenzoylisocyanat und 14,0 g 3-Chlor-4-(4-chlorphenoxy)phenylisocyanat werden mit 3 Tropfen Triethylamin 5 h bei 100°C gerührt. Es wird gründlich mit Petrolether gewaschen und getrocknet; 16 g 2-(2,6-Difluorphenyl)-5-[3-chlor-4-(4-chlorphenoxy)phenyl]-4H-5,6-dihydro-1,3,5-oxadiazin-4,6-dion vom Schmelzpunkt 180 bis 185°C (in der nachstehenden Tabelle der Nr. 1 entsprechend).

b) 4,76 g 2,6-Difluorbenzoylisocyanat und 6,27 g 4-(3-Trifluormethylphenoxy)phenylisocyanat wurden mit 0,2 ml $PCl_3$ 2½ Stunden bei 70°C und 2 Stunden bei 110°C gerührt. Man erhielt 11 g 2-(2,6-Difluorphenyl)-5-[4-(3-trifluormethylphenoxy)phenyl]-4H-5,6-dihydro-1,3,5-oxadiazin-4,6-dion. Die Verbindung wurde mit Ether gewaschen und aus Toluol/Cyclohexan umkristallisiert und wies danach einen Schmelzpunkt von 127 bis 129°C auf.

c) 3,66 g 2,6-Difluorbenzoylisocyanat, 5,8 g 4-(4-Bromphenoxy)phenylisocyanat und 2 Tropfen Dibutylzinndiacetat wurden 2 Stunden bei 50°C, 4 Stunden bei 100°C, 8 Stunden bei 120°C gerührt. Man erhielt 9 g 2-(2,6-Difluorphenyl)-5-[4-(4-bromphenoxy)phenyl]-4H-5,6-dihydro-1,3,5-oxadiazin-4,6-dion. Die Verbindung wurde mit Ether gewaschen und wies danach einen Schmelzpunkt von 186 bis 188°C auf.

Die in der nachstehenden Tabelle 1 mit ihren Schmelzpunkten angegebenen Stoffe wurden nach den vorstehenden Herstellangaben erhalten; dabei beziehen sich die Tabellennummern 1, 2 und 3 auf die Herstellungsbeispiele a), b) bzw. c).

Weitere typische Verbindungen können nach der Aufgliederung in der Tabelle 2 erhalten werden; ihre Wirksamkeit kann aufgrund der strukturellen Ähnlichkeit mit den untersuchten Stoffen erwartet werden.

Tabelle 1

| Nr. | $R^1$ | $R^2$ | $R^3$ | $R^4$ | $R^5$ | $R^6$ | $R^7$ | $R^8$ | Fp. °C |
|---|---|---|---|---|---|---|---|---|---|
| 1 | F | F | H | Cl | H | H | Cl | H | 180–185 |
| 2 | F | F | H | H | H | H | $CF_3$ | H | 127–129 |
| 3 | F | F | H | H | H | H | Br | H | 186–188 |
| 4 | Cl | Cl | H | Cl | H | H | Cl | H | 196–199 |
| 5 | Br | H | H | Cl | H | H | Cl | H | 185–187 |
| 6 | $CH_3$ | H | H | Cl | H | H | Cl | H | 165–168 |
| 7 | Cl | Cl | H | H | H | H | Br | H | 185–188 |
| 8 | F | F | H | Cl | H | H | $OCHF_2$ | H | 169–173 |
| 9 | Cl | Cl | H | Cl | H | H | $OCHF_2$ | H | 200–204 |

Tabelle 1 (Fortsetzung)

| Nr. | $R^1$ | $R^2$ | $R^3$ | $R^4$ | $R^5$ | $R^6$ | $R^7$ | $R^8$ | Fp. °C |
|---|---|---|---|---|---|---|---|---|---|
| 10 | Cl | H | H | Cl | H | H | $OCHF_2$ | H | 149–151 |
| 11 | F | F | H | H | H | H | $OCHF_2$ | H | 168–172 |
| 12 | Cl | H | H | H | H | H | $OCHF_2$ | H | 179–182 |
| 13 | Cl | Cl | H | F | H | H | Br | H | 191–195 |
| 14 | Cl | H | H | H | H | $CF_3$ | H | H | 155–159 |
| 15 | F | F | Cl | H | H | H | H | Cl | 140–144 |
| 16 | Cl | Cl | Cl | H | H | H | H | Cl | 179–181 |
| 17 | Cl | H | Cl | H | H | H | H | Cl | 167–176 |
| 18 | F | F | H | H | H | H | Cl | H | 173–178 |
| 19 | Cl | Cl | H | H | H | H | Cl | H | 169–174 |
| 20 | Cl | H | H | H | H | H | Cl | H | 172–176 |

Tabelle 2

| Nr. | $R^1$ | $R^2$ | $R^3$ | $R^4$ | $R^5$ | $R^6$ | $R^7$ | $R^8$ |
|---|---|---|---|---|---|---|---|---|
| 21 | F | F | H | Cl | H | H | H | H |
| 22 | F | F | H | Cl | H | H | F | H |
| 23 | Cl | Cl | H | Cl | H | H | F | H |
| 24 | F | Cl | H | Cl | H | H | Cl | H |
| 25 | Cl | H | H | Cl | H | H | Cl | H |
| 26 | F | Cl | H | H | H | H | Br | H |
| 27 | Cl | H | H | H | H | H | Br | H |
| 28 | F | Cl | H | Cl | H | H | $OCHF_2$ | H |
| 29 | Cl | Cl | H | H | H | H | $OCHF_2$ | H |
| 30 | F | Cl | H | H | H | H | $OCHF_2$ | H |
| 31 | F | F | H | Cl | H | H | $CF_3$ | H |
| 32 | F | F | H | Cl | H | H | $OCF_3$ | H |
| 33 | F | F | H | Cl | H | H | $OCH_3$ | H |
| 34 | F | F | H | F | H | H | Cl | H |
| 35 | Cl | Cl | H | F | H | H | Cl | H |
| 36 | F | Cl | H | F | H | H | Cl | H |
| 37 | Cl | H | H | F | H | H | Cl | H |

Tabelle 2 (Fortsetzung)

| Nr. | $R^1$ | $R^2$ | $R^3$ | $R^4$ | $R^5$ | $R^6$ | $R^7$ | $R^8$ |
|---|---|---|---|---|---|---|---|---|
| 38 | F | F | H | F | H | H | Br | H |
| 39 | F | Cl | H | F | H | H | Br | H |
| 40 | Cl | H | H | F | H | H | Br | H |
| 41 | Br | H | H | F | H | H | Br | H |
| 42 | $CH_3$ | H | H | F | H | H | Br | H |
| 43 | Cl | H | H | F | H | H | $CF_3$ | H |
| 44 | F | F | H | F | H | H | $OCF_3$ | H |
| 45 | F | F | H | F | H | H | $OCH_3$ | H |
| 46 | F | F | H | H | H | H | $CH_3$ | H |
| 47 | Cl | H | H | F | H | H | $OC_2H_5$ | H |
| 48 | F | F | H | H | H | H | $OCF_2CHClF$ | H |
| 49 | Cl | H | Cl | Cl | H | H | F | H |
| 50 | F | F | Cl | Cl | H | H | Cl | H |
| 51 | F | F | Cl | Cl | H | H | H | H |
| 52 | Cl | Cl | Cl | Cl | H | H | H | H |
| 53 | F | Cl | Cl | Cl | H | H | H | H |
| 54 | Cl | H | Cl | Cl | H | H | H | H |
| 55 | F | F | Br | Br | H | H | H | H |
| 56 | F | F | Br | H | H | H | Cl | H |
| 57 | F | F | $CF_3$ | H | H | H | Cl | H |
| 58 | Cl | Cl | $CF_3$ | H | H | H | Cl | H |
| 59 | F | Cl | $CF_3$ | H | H | H | Cl | H |
| 60 | Cl | H | $CF_3$ | H | H | H | Cl | H |
| 61 | F | F | $CH_3$ | H | H | H | Cl | H |
| 62 | Cl | Cl | H | H | H | $CF_3$ | H | H |
| 63 | F | Cl | H | H | H | $CF_3$ | H | H |
| 64 | F | F | H | H | H | Cl | H | H |
| 65 | F | Cl | Cl | H | H | H | H | Cl |
| 66 | F | F | H | H | Cl | H | Cl | H |
| 67 | Cl | Cl | H | H | Cl | H | Cl | H |

Tabelle 2 (Fortsetzung)

| Nr. | $R^1$ | $R^2$ | $R^3$ | $R^4$ | $R^5$ | $R^6$ | $R^7$ | $R^8$ |
|---|---|---|---|---|---|---|---|---|
| 68 | F | Cl | H | H | Cl | H | Cl | H |
| 69 | Cl | H | H | H | Cl | H | Cl | H |
| 70 | F | F | H | H | Cl | H | $CF_3$ | H |
| 71 | F | Cl | H | H | H | H | Cl | H |

Die Diphenyloxadiazindione der allgemeinen Formel I bzw. hieraus hergestellte Mittel sind geeignet, Schädlinge vor allem aus der Klasse der Insekten und Spinnmilben wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise

Plutella maculipennis (Kohlschabe), Leucoptera coffeella (Kaffeemotte), Hyponomeuta malinellus (Apfelbaumgespinstmotte), Argryresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria Mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarus (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamme (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise

Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varicestris (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);

aus der Ordnung der Zweiflügler (Diptera) beispielsweise

Lycoria pectoralis, Mayetiola destructor (Hessenfliege), Dasineura brassicae (Kohlschoten-Gallmücke), Contarinia tritici (Gelbe Weizen-Gallmücke), Haplodiplosis equestris (Sattelmücke),

Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina moristans (Tsetse-Fliege), Oestrus ocis, Chrysomya macellaria, Chrysomya hominivorax, Lucilia cuprina, Lucilia sericata, Hypoderma lineata;

aus der Ordnung der Hautflügler (Hymenoptera) beispielsweise

Athalia rosae (Rübenblattwespe), Hoplocampa minuta (Pflaumensägewespe), Monomorium pharaonis (Pharaoameise), Solenopsis geminata (Feuerameise), atta sexdens (Blattschneiderameise);

aus der Ordnung der Wanzen (Heteroptera) beispielsweise

Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise

Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nastrutii (Kreuzdornblattlaus), Cerosipha gossypii (Gurkenblattlaus), Sappaphis mali (Rosige Apfellaus), Sappaphis mala (Mehlige Birnblattlaus), Dysphis radicola (Mehlige Apfelfalterlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (schwarze Sauerkirschenlaus), Dysaulacorthum pseudosolani (Gefleckte Kartoffellaus), Acyrthosiphon onobrychis (Grüne Erbsenlaus), Macrosiphon rasae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);

aus der Ordnung der Termiten (Isoptera) beispielsweise

Reticulitermes lucifugus, Calotermes flavicollis, Leucotermes flavipes, Termes natalensis;

aus der Ordnung der Geradflügler (Orthoptera) beispielsweise

Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melano plus femur-rubrum (Rotbeinige Heuschrecke), Blatta orientalis (Küchenschabe), Blattella germanica (Deutsche Schabe), Periplaneta americana (Amerikanische Schabe), Blabera gigantea (Riesenschabe).

Die Mittel können auch verwendet werden zur Bekämpfung z. B. von Spinnentieren (Arachnoidea bzw. Acarina) wie Ixodes ricinus (Holzbock), Ornithodorus moubata, Amblyomma americanum, Dermacentor silvarum, Boophilus microplus, Tetranychus telarius, Tetranychus atlanticus, Tetranychus pacificus, Paratetranychus pilosus, Bryobia praetiosa; ferner zur Bekämpfung von Fadenwürmern (Nemathelminthes), wie Wurzelgallennematoden, z. B. Meloidogyne incognita, Meloidogyne hapla, Meloidogyne javanica, Zysten bildende Nematoden, z. B. Heterodera rostochiensis, Heterodera schachtii, Heterodera avenae, Heterodera glycines, Heterodera triflolii, Stock- und Blattälchen, z. B. Ditylenchus dipsaci, Ditylenchus destructor, Paratylenchus neglectus, Paratylenchus penetrans, Paratylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsmöglichkeiten, z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gewichtsteile der Verbindung Nr. 1 werden mit 95 Gewichtsteilen feinteiligem Kaolin innig vermischt. Man erhält auf diese Weise ein Stäubemittel, das 5 Gew.-% des Wirkstoffs enthält.

II. 30 Gewichtsteile der Verbindung Nr. 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gewichtsteile der Verbindung Nr. 4 werden in einer Mischung gelöst, die aus 90 Gewichtsteilen Xylol, 6 Gewichtsteilen des Anlagerungsproduktes von 8 bis 10 Mol Äthylenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

IV. 20 Gewichtsteile der Verbindung Nr. 13 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

V. 80 Gewichtsteile der Verbindung Nr. 19 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden.

Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,2 bis 10, vorzugsweise 0,5 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden:

1,2-Dibrom-3-chlorpropan, 1,3-Dichlorpropen, 1,3-Dichlorpropen + 1,2-Dichlorpropan, 1,2-Dibrom-ethan, 2-sec.-Butyl-phenyl-N-methylcarbamat, o-Chlorphenyl-N-methylcarbamat, 3-Isopropyl-5-methylphenyl-N-methylcarbamat, o-Isopropoxyphenyl-N-methylcarbamat, 3,5-Dimethyl-4-methylmercapto-phenyl-N-methylcarbamat, 4-Dimethylamino-3,5-xylyl-N-methyl-carbamat, 2-(1,3-Dioxolan-2-yl)-phenyl-N-methylcarbamat, 1-Naphthyl-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-O-(methylcarbamoyl)-oxim, S-Methyl-N-[(methylcarbamoyl)-oxy]-thio-acetimidat, Methyl-N',N'-dimethyl-N-[(methylcarbamoyl)oxy]-1-thiooxamidat, N-(2-Methyl-chlor-phenyl)-N',N'-dimethylformamidin, Tetrachlorthiophen, 1-(2,6-Difluor-benzoyl)-3-(4-chlor-phenyl)-harnstoff, O,O-Dimethyl-O-(p-nitrophenyl)-phosphorthioat, O,O-Diethyl-O-(p-nitrophenyl)-phosphorthioat, O-Ethyl-O-(p-nitrophenyl)-phenyl-phosphonothioat, O,O-Dimethyl-O(3-methyl-4-nitrophenyl)-phosphorthioat, O,O-Diethyl-O-(2,4-dichlorphenyl)-phosphorthioat, O-Ethyl-O-(2,4-dichlorphenyl)-phenylphosphonothioat, O,O-Dimethyl-O-(2,4,5-trichlorphenyl)-phosphorthioat, O-Ethyl-O-(2,4,5-trichlorphenyl)-ethyl-phosphonothioat, O,O-Dimethyl-O-(4-brom-2,5-dichlorphenyl)-phosphorthioat, O,O-Dimethyl-O-(2,5-dichlor-4-jodphenyl)-phosphorthioat, O,O-Dimethyl-O-(3-methyl-4-methylthiophenyl)-phosphorthioat, O-Ethyl-O-(3-methyl-4-methylthiophenyl)-isopropyl-phosphoramidat, O,O-Diethyl-O-[p-methylsulfinyl-phenyl]-phosphorthioat, O-Ethyl-S-phenyl-ethyl-phosphonodithioat, O,O-Diethyl-[2-chlor-1-(2,4-dichlorphenyl)-vinyl]-phosphat, O,O-Dimethyl-[2-chlor-1-(2,4,5-trichlorphenyl)]-vinyl-phosphat, O,O-Dimethyl-S-1'-phenyl)-ethylacetat-phosphordithioat,Bis-(dimethylamino)-fluorphosphinoxid, Octamethyl-pyrophosphoramid, O,O,O,O-Tetraethyldithio-pyrophosphat, S-Chlormethyl-O,O-diethyl-phosphordithioat, O-Ethyl-S,S-dipropyl-phosphordithioat, O,O-Dimethyl-O-2,2-dichlorvinyl-phosphat, O,O-Dimethyl-1,2-dibrom-2,2-dichlorethylphosphat, O,O-Dimethyl-2,2,2-trichlor-1-hydroxy-ethylphosphonat, O,O-Dimethyl-S-[1,2-biscarbethoxy-ethyl-(1)]-phosphordithioat, O,O-Dimethyl-O-(1-methyl-2-carbmethoxy-vinyl)-phosphat, O,O-Dimethyl-S-(N-methyl-carbamoyl-methyl)-phosphordithioat, O,O-Dimethyl-S-(N-methyl-carbamoyl-methyl)-phosphorthioat, O,O-Dimethyl-S-(N-methoxyethyl-carbamoyl-methyl)-phosphordithioat, O,O-Dimethyl-S-(N-methoxyethyl-carbamoyl-methyl)-phosphordithioat, O,O-Dimethyl-S-(N-formyl-N-methyl-carbamoylmethyl-phosphordithioat, O,O-Dimethyl-O-[1-methyl-2-(methyl-carbamoyl)-vinyl]-phosphat, O,O-Dimethyl-O-[(1-methyl-2-dimethylcarbamoyl)-vinyl]-phosphat, O,O-Dimethyl-O-[(1-methyl-2-chlor-2-diethylcarbamoyl)-vinyl]-phosphat, O,O-Diethyl-S-(ethyl-thio-methyl)-phosphordithioat, O,O-Diethyl-S-[(p-chlor-phenylthio)-methyl]-phosphordithioat, O,O-Dimethyl-S-(2-ethylthioethyl)-phosphorthioat, O,O-Dimethyl-S-(2-ethylthioethyl)-phosphordithioat, O,O-Dimethyl-S-(2-ethylsulfinyl-ethyl)-phosphorthioat, O,O-Diethyl-S-(2-ethylthio-ethyl)-phosphordithioat, O,O-Diethyl-S-2-ethylsulfinyl-ethyl)-phosphorthioat, O,O-Diethyl-thio-phosphoryliminophenyl-acetonitril, O,O-Diethyl-S-(2-chlor-1-phthalimidoethyl)-phosphordithioat, O,O-Diethyl-S-[6-chlor-benzoxazolon-(2)-yl-(3)]-methyldithiophosphat, O,O-Dimethyl-S-[2-methoxy-1,3,4-thiadiazol-5-[4H]-onyl-(4)-methyl]-phosphordithioat, O,O-Diethyl-O-[3,5,6-trichlor-pyridyl-(2)]-phosphorthioat, O,O-Diethyl-O-(2-pyrazinyl)-phosphorthioat, O,O-Diethyl-O-[2-isopropyl-4-methyl-pyrimidinyl-(6)]-phosphorthioat, O,O-Diethyl-O-[2-(diethylamino)-6-methyl-4-pyrimidinyl]-thionophosphat, O,O-Dimethyl-S-(4-oxo-1,2,3-benzotriazin-3-[4H]-yl-methyl)-phosphordithioat, O,O-Dimethyl-S-[(4,6-diamino-1,3,5-triazin-2-yl)-methyl]-phosphordithioat, O,O-Diethyl-(1-phenyl-1,2,4-triazol-3-yl)-thionophosphat, O,S-Dimethyl-phosphor-amino-thioat, O,S-Dimethyl-N-acetyl-phosphoramidothioat, $\gamma$-Hexachlorcyclohexan, 1,1-Di-(p-methoxyphenyl)-2,2,2-trichlor-ethan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on-(1)-yl-(4)-DL-cis,trans-chrysanthemat, 5-Benzyl-furyl-(3)-methyl-DL-cis,trans-chrysanthemat, 3-Phenoxybenzyl(±)-cis,trans-2,2-dimethyl-3-(2,2-dichlorvinyl)-cyclopropancarboxylat, $\alpha$-Cyano-3-phenoxybenzyl(±)-cis,trans-2,2-dimethyl-3-(2,2-dichlorvinyl)-cyclopropancarboxylat, (s)-$\alpha$-Cyano-3-phenoxybenzyl-cis(1R,3R)-2,2-dimethyl-3-(2,2-dibromvinyl)-cyclopropancarboxylat, 3,4,5,6-Tetrahydrophthalimidoethyl-DL-cis, trans-chrysanthemat, 2-Methyl-5-(2-propinyl)-3-furylmethyl-chrysanthemat, (α-Cyano-3-phenoxy-benzyl)-α-isopropyl-4-chlorphenylacetat.

## Anwendungsbeispiele

Für die nachfolgenden Versuche wurde die Verbindung 1 der Tabelle 1 und als Vergleichsmittel das aus der DE-OS 2 732 115 bekannte 2-(2,6-Difluorphenyl)-5-(3,4-dichlorphenyl)-4H,5,6-dihydro-1,3,5-oxadiazin-4,6-dion verwendet.

Außerdem konnte gezeigt werden, daß die erfindungsgemäße Verbindung gegenüber einem typischen, handelsüblichen Benzoylharnstoffderivat, dem Diflubenzuron, eine deutlich höhere Wirksamkeit bei der Bekämpfung von Stechmücken (Moskito)larven aufweist.

### 1. Zuchtversuch mit Stubenfliegen (Musca domestica)

Je 4,5 ml Magermilch füllt man in 50 ml Penicillingläser und versetzt mit 0,5 ml der wäßrigen Wirkstoffaufbereitung. Nach kurzem Mischen fügt man ein Wattebällchen dazu und belegt dieses mit ca. 50 Eilarven der Stubenfliege. Die Gläser lagert man abgedeckt bei Raumtemperatur und beurteilt die Entwicklung nach 7 Tagen.

Ergebnis:

| | | |
|---|---|---|
| Beanspruchte Verbindung | 2,5 ppm | 100% Mort. |
| Vergleichsmittel | 50,0 ppm | 100% Mort. |
| | 25,0 ppm | 60% Mort. |

### 2. Zuchtversuch mit Moskitolarven (Aedes aegypti)

200 ml Leitungswasser werden mit der Wirkstoffaufbereitung versetzt und darauf mit 20 bis 30 Moskitolarven im 3. bis 4. Larvenstadium besetzt. Die Gefäße stehen bei 25°C. Beobachtet werden Verpuppung und Schlüpfen der Imagines, welches nach 10 bis 12 Tagen erfolgt. Während der Beobachtungszeit wird einmal mit einem gepulverten Aquarienfischfutter gefüttert.

Ergebnis:

| | | |
|---|---|---|
| Beanspruchte Verbindung | 0,001 ppm | 100% Mort. |
| Vergleichsmittel | 0,25 ppm | 100% Mort. |
| | 0,01 ppm | unwirksam |
| Diflubenzuron | 0,004 ppm | 100% Mort. |
| | 0,002 ppm | 60% Mort. |
| | 0,001 ppm | 20% Mort. |

### 3. Zuchtversuch mit Baumwollwanzen (Dysdercus intermedius)

Die Wanzen werden im 4. Larvenstadium in Petrischalen (Ø 10 cm) dem Wirkstoffbelag der Testsubstanz für 24 Stunden zugesetzt. Die Überlebenden züchtet man in 1-1-Gläsern auf feuchtem Quarzsand, der vorher mit der Wirkstofflösung versetzt wurde bis zum Schlüpfen der $F_1$-Generation.

Dabei entsprechen in der Behandlung:

| | |
|---|---|
| 2,5 mg/Schale | 25 ppm im Sand |
| 1,0 mg/Schale | 10 ppm im Sand |
| 0,5 mg/Schale | 5 ppm im Sand |
| usw. | |

Beurteilt wird Mortalität und Vermehrung.

Ergebnis:

| | | |
|---|---|---|
| Beanspruchte Verbindung | 25 ppm | 100% Mort. |
| Vergleichsmittel | 25 ppm | unwirksam |

4. Wirkung auf Spinnmilben (Tetranychus telarius)

Getopfte Buschbohnen, die das erste Folgeblatt entwickelt haben und einen starken Besatz aller Stadien der Spinnmilbe Tetranychus telarius tragen, werden in der Spritzkabine mit der wäßrigen Wirkstoffaufbereitung tropfnaß gespritzt. Die Pflanzen kommen dazu auf einen Drehteller und werden von allen Seiten mit je 50 ml Spritzbrühe besprüht. Der Sprühvorgang dauert ca. 22 Sekunden. Nach 8 Tagen werden die Pflanzen auf lebende Spinnmilben untersucht.

Ergebnis:

| | | |
|---|---|---|
| Beanspruchte Verbindung | 0,05% | 100% Mort. |
| Vergleichsmittel | 0,1% | 60% Mort. |

**Patentansprüche**

1. Oxadiazindione der allgemeinen Formel I

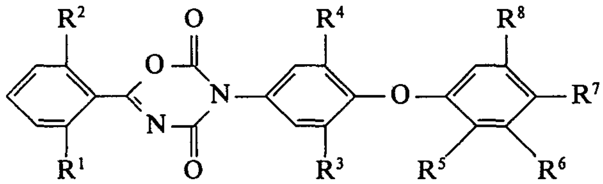

(I)

in der

$R^1$ für $CH_3$, F, Cl, Br und
$R^2$ für H, F, Cl und
$R^3$ bis $R^8$ für H, F, Cl, Br, $CH_3$, $CF_3$, $-OCF_3$, $-OCHF_2$, $-OCF_2CHClF$, $-OCH_3$ oder $-OC_2H_5$ stehen.

2. Verfahren zur Herstellung von Oxadiazindionen gemäß Anspruch 1, dadurch gekennzeichnet, daß man ein Benzoylisocyanat der allgemeinen Formel II

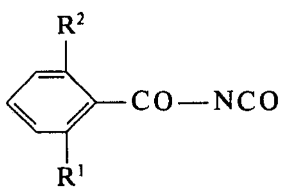

(II)

in der $R^1$ und $R^2$ die im Anspruch 1 genannten Bedeutungen haben, mit einem Isocyanat der allgemeinen Formel III

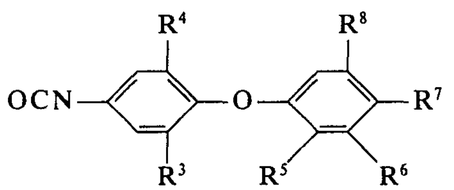

(III)

in der $R^3$ bis $R^8$ die im Anspruch 1 genannten Bedeutungen haben, bei einer Temperatur unterhalb von 160°C umsetzt.

3. Insektizides und akarizides Mittel, enthaltend ein Oxadiazindion gemäß Anspruch 1.

4. Insektizides und akarizides Mittel, enthaltend inerte Zusatzstoffe und ein Oxadiazindion gemäß Anspruch 1.

5. Verwendung von Oxadiazindionen gemäß Anspruch 1 zur Bekämpfung von Insekten und Spinnentieren.

6. Verfahren zur Bekämpfung von Insekten und Spinnentieren, dadurch gekennzeichnet, daß man eine wirksame Menge eines Oxadiazindions gemäß Anspruch 1 auf Insekten oder Spinnentiere und/oder deren Lebensraum einwirken läßt.

7. Verfahren zur Herstellung eines insektiziden und akariziden Mittels, dadurch gekennzeichnet, daß man ein Oxadiazindion gemäß Anspruch 1 mit oberflächenaktiven Verbindungen und/oder Streckmitteln und/oder anderen Wirkstoffen mischt.

**Claims**

1. An oxadiazinedione of the general formula I

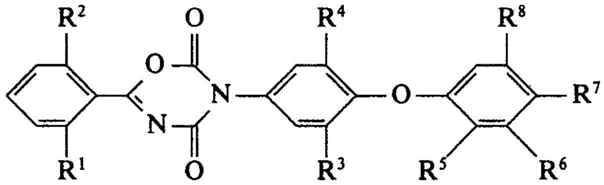

(I)

where $R^1$ is $CH_3$, F, Cl or Br, $R^2$ is H, F or Cl and $R^3$ to $R^8$ are H, F, Cl, Br, $CH_3$, $CF_3$, $-OCF_3$, $-OCHF_2$, $-OCF_2CHClF$, $-OCH_3$ or $-OC_2H_5$.

2. A process for manufacturing an oxadiazinedione as claimed in claim 1, wherein a benzoyl isocyanate of the general formula II

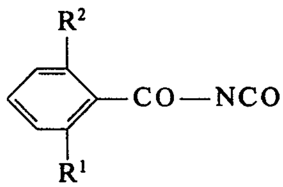

(II)

where $R^1$ and $R^2$ have the meanings given in claim 1, is reacted with an isocyanate of the general formula III

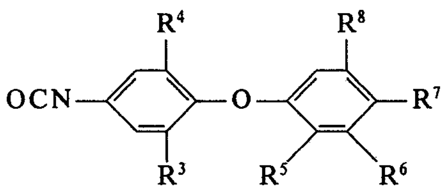

(III)

where $R^3$ to $R^8$ have the meanings given in claim 1, at a temperatur below 160°C.

3. An insecticidal and acaricidal agent containing an oxadiazinedione as claimed in claim 1.

4. An insecticidal and acaricidal agent containing inert additives and an oxadiazinedione as claimed in claim 1.

5. The use of oxadiazinediones as claimed in claim 1 for combating insects and Arachnida.

6. A process for combating insects and Arachnida, wherein an effective amount of an oxadiazinedione as claimed in claim 1 is allowed to act on the insects or Arachnida, and/or their habitat.

7. A process for preparing an insecticidal and acaricidal agent, wherein an oxadiazinedione as claimed in claim 1 is mixed with surfactants and/or extenders and/or other active ingredients.

**Revendications**

1. Oxadiazindiones de formule générale I

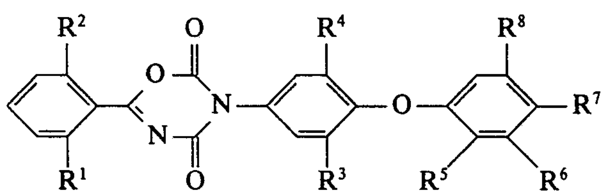

(I)

dans laquelle

| | |
|---|---|
| $R^1$ | représente $CH_3$, F, Cl, Br et |
| $R^2$ | H, F, Cl et |
| $R^3$ à $R^8$ | H, F, Cl, Br, $CH_3$, $CF_3$, $-OCF_3$, $-OCHF_2$, $-OCF_2CHClF$, $-OCH_3$ ou $-OC_2H_5$. |

2. Procédé de préparation d'oxadiazindiones selon la revendication 1, caractérisé par le fait que l'on fait réagir, à une température inférieure à 160°C, un isocyanate de benzoyle de formule générale II

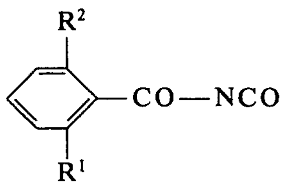

(II)

dans laquelle $R^1$ et $R^2$ ont les significations indiquées dans la revendication 1, avec un isocyanate de formule générale III

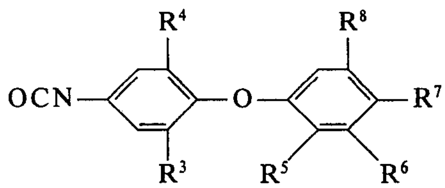

(III)

dans laquelle $R^3$ à $R^8$ ont les significations indiquées dans la revendication 1.

3. Insecticide et agent acaricide, contenant une oxadiazindione selon la revendication 1.

4. Insecticide et agent acaricide, contenant des additifs inertes et une oxadiazindione selon la revendication 1.

5. Utilisation d'oxadiazindiones selon la revendication 1, pour la lutte contre les insectes et les arachnides.

6. Procédé pour lutter contre les insectes et les arachnides, caractérisé par le fait que l'on fait agir une quantité efficace d'une oxadiazindione selon la revendication 1 sur des insectes ou des arachnides et/ou sur leur biotope.

7. Procédé de préparation d'un insecticide et agent acaricide, caractérisé par le fait qu'on mélange une oxadiazindione selon la revendication 1 avec des composés tensio-actifs et/ou extendeurs et/ou autres principes actifs.